# EUROPEAN PATENT APPLICATION

(11) **EP 1 152 057 A1**
(43) Date of publication of application: **07.11.2001**
(21) Application number: 00979079.1
(22) Date of filing: 04.12.2000
(51) Int. Cl.: C12N 15/06, C12N 5/10, C07K 16/40, C12Q 1/37, A61K 39/395, A61P 11/00, A61P 11/04, A61P 11/06, A61P 11/10, A61P 31/12, A61P 35/00, A61P 35/04, G01N 33/573, G01N 33/577

(54) **ANTIBODY BINDING TO HUMAN AIRWAY TRYPSIN-LIKE ENZYME AND UTILIZATION THEREOF**

(30) Priority: 06.12.1999 JP 34589099
(71) Applicant: TEIJIN LIMITED, Osaka-shi Osaka 541-0054 (JP)
(72) Inventor: EGUCHI, Hiroshi, Teijin Limited, Hino-shi, Tokyo 191-0065 (JP); YAMAOKA, Kazuyoshi, Teijin Limited, Hino-shi, Tokyo 191-0065 (JP); OGAWA, Hiroko, Teijin Limited, Hino-shi, Tokyo 191-0065 (JP); YASUOKA, Susumu, School of Medical Science, Tokushima-shi, Tokushima 770-0042 (JP)
(74) Representative: Hallybone, Huw George
(86) International application number: JP0008579
(87) International publication number: WO0142450

(57) **Abstract**

Problems of the present invention are to provide methods for diagnosing the morbid state based on the assay of a human airway trypsin-like protease in a biosample and to provide a therapeutic agent for diseases associated with the human airway trypsin-like protease.

Such problems are solved with antibodies binding to the human airway trypsin-like protease and an enzyme immunoassay using the antibodies. Furthermore, the problems are solved with a therapeutic agent comprising the antibodies inhibiting the activation of the human airway trypsin-like protease and a therapeutic agent comprising the antibodies neutralizing the human airway trypsin-like protease activity.

SEQUENCE LISTING
<110>TEIJIN LIMITED
EGUCHI, Hiroshi
YAMAOKA, Kazuyoshi
OGAWA, Hiroko
YASUOKA, Susumu
<120>ANTIBODIES BINDING TO HUMAN AIRWAY TRYPSIN-LIKE
PROTEASE AND METHODS FOR UTILIZING THE SAME
<130>T-419
<150>JP P1999-345890
<151>1999-12-6
<160>1
<210>1
<211>20
<212>PRT
<213>Homo sapiens
<400>1
Ile Leu Gly Gly Thr Glu Ala Glu Glu Gly Ser Trp Pro Trp Gln Val Ser Leu
1               5                   10                  15
Arg Leu
    20

## Description

### Technical Field

This invention relates to antibodies binding to a human airway trypsin-like protease, a detecting or an assaying system for the human airway trypsin-like protease using the antibodies, a diagnostic agent using the same and a therapeutic agent using the same.

### Background Art

A Human Airway Trypsin-like Protease hereinafter referred to as HAT has recently been purified from the sputum of patients suffering from human chronic airway inflammations (see Japanese Unexamined Patent Publication No. 7-067640 and S. Yasuoka et al., Am. J. Respir. Cell Mol. Biol., 16: pp. 300-308, 1997), and the amino acid sequence and cDNA sequence thereof have already been made clear [see Japanese Unexamined Patent Publication No. 8-89246, US Patent 5804410, EP 699763 and Yamaoka K. et al., J. Biol. Chem., 273 (19), 11895-11901, 1998]. Several studies have been made of the activity possessed by the enzyme in vitro. Since the enzyme has production enhancing actions on cytokines such as IL-8 or GM-CSF derived from a human bronchial epithelial cell line including the association with mucociliary movement (Terao, Noriko et al., the Japanese Respiratory Society, 1998), the possibility for association with the morbid state of airway inflammations is considered. Furthermore, since the enzyme has enzyme activities such as hydrolytic activity for fibrinogen and activating actions on a plasminogen activator (pro-urokinase) (Yoshinaga, Junko et al., Conference on Proteases and Inhibitors in Pathophysiology and Therapeutics, 1998), the possibility is assumed for antiinflammatory actions through the formation of fibrins on the airway mucosal surfaces or modification of the morbid state thereof in chronic airway diseases and the possibility is also considered for the association with cancer metastasis or the like.

### Disclosure of the Invention

Problems of the present invention are to obtain antibodies for detecting the above HAT and the construction of the assaying system using the antibodies. Accordingly, the concentration of the HAT in vivo can be assayed and the distribution of the HAT in tissues or cells can be studied to thereby carry out the diagnosis of inflammatory diseases, cancers or the like. To obtain antibodies for neutralizing the HAT activity and antibodies for inhibiting the activation of the HAT is also problems of the present invention. The antibodies for neutralizing the HAT activity or the antibodies inhibiting the activation of the HAT suppress or modify physiological actions such as actions of the HAT on the coagulating and fibrinolytic system, actions on airway remodeling, actions on airway inflammations, actions on the proliferation or metastasis of cancers, actions on mucociliary movement or antiviral infectious actions and can be used for ameliorating or treating the morbid state. Further, the antibodies provide a new therapeutic agent or diagnostic agent for diseases such as chronic airway inflammations [chronic obstructive pulmonary disease (COPD) or asthma], abnormality in coagulation and fibrinolysis or cancers.

The present inventors et al. have obtained a monoclonal antibody and a polyclonal antibody binding to the HAT, have succeeded in detecting the HAT in vivo by using the monoclonal and polyclonal antibodies and have further found the HAT neutralizing activity in the obtained antibodies, thus accomplishing the present invention.

The present invention is antibodies binding to the human airway trypsin-like protease. Furthermore, the present invention includes antibodies capable of assaying the natural human airway trypsin-like protease in vivo by an enzyme immunoassay using the antibodies or antibodies capable of detecting the natural human airway trypsin-like protease in vivo by a western blotting technique. The present invention is antibodies in which the epitope is present between the 187th and the 418th positions of the amino acid sequence of the human airway trypsin-like protease.

The "human airway trypsin-like protease" herein refers to a protein including a part or all of the cDNA sequence of the human airway trypsin-like protease disclosed in Japanese Unexamined Patent Publication No. 8-89246. The amino acid sequence numbers of the human airway trypsin-like protease conform to the amino acid sequence numbers disclosed in Yamaoka K. et al., J. Biol. Chem., 273(19), 11895-11901, 1998.

In addition, the present invention is a method for detecting or assaying the human airway trypsin-like protease using the antibodies binding to the human airway trypsin-like protease and an enzyme immunoassay for the human airway trypsin-like protease using the antibodies.

The present invention is a therapeutic agent comprising the antibodies inhibiting the activation of the human airway trypsin-like protease and a therapeutic agent comprising the antibodies neutralizing the human airway trypsin-like protease activity.

Furthermore, the present invention is an animal cell producing the antibodies binding to the human airway trypsin-like protease.

### Brief Description of Drawings

Fig. 1 illustrates an evaluation of the neutralizing activity of the anti-HAT antibody of the present invention.

Fig. 2 illustrates a standard curve of the assaying system using the anti-HAT polyclonal antibody of the present invention for a recombinant HAT.

Fig. 3 illustrates results of assay of the HAT in the sputm by the assaying system using the anti-HAT polyclonal antibody of the present invention.

Fig. 4 illustrates the results of examination on the liquid-phase antigenic reactivity of the anti-HAT monoclonal antibody and the anti-HAT polyclonal antibody of the present invention toward the recombinant HAT.

Fig. 5 illustrates curves of the assaying system using the anti-HAT monoclonal antibody and the anti-HAT polyclonal antibody of the present invention for the recombinant HAT.

### Best Mode for Carrying Out the Invention

The polyclonal antibody of the present invention can be obtained by, for example, the following method: That is, the HAT which is an antigenic protein may be obtained by purification from a biosample such as in the sputum or an HAT-producing cultured cell according to the method disclosed in Japanese Unexamined Patent Publication No. 7-067640 and S. Yasuoka et al., Am. J. Respir. Cell Mol. Biol., 16, pp. 300-308, 1997 or by producing the recombinant HAT and purifying the resulting recombinant HAT according to the method disclosed in Japanese Unexamined Patent Publication No. 8-89246, US Patent 5804410, EP 699763 and Yamaoka K. et al., J. Biol. Chem., 273 (19), 11895-11901, 1998.

The intraperitoneal or subcutaneous injection of the purified HAT together with an adjuvant into an animal is then repeated to cause an immune response in the animal. Mammals are preferred as the animal used, and rabbits, goats, sheep, guinea pigs, hamsters, rats and mice are more preferred. Rabbits are most preferred.

Blood can subsequently be collected from the immunized animal to separate serum components, and IgG can be purified with a protein A column according to a conventional method to obtain the anti-HAT polyclonal antibody.

The monoclonal antibody of the present invention can be prepared by, for example, the following method: That is, the method is fundamentally that described in Galfre and Milstein, Methods in Enzymology, vol. 73, Academic Press, New York (1981); James W. Goding, Monoclonal Antibodies: Principles and Practice, Academic Press, Orlando, Florida (1986); Current Protcols in Molecular Biology, jointly edited by F. M. Ausubel et al., Wiley Interscience, New York (1987) or the like.

The antigenic protein is preferably purified in order to prepare the monoclonal antibody. The purified protein can also be used for antibody potency tests. The method for expressing and purifying the recombinant HAT used in preparing the monoclonal body of the present invention is detailed in Japanese Unexamined Patent Publication No. 8-89246.

The intraperitoneal or subcutaneous injection of the purified recombinant HAT together with an adjuvant into an animal is then repeated to cause an immune response in the animal. Mammals are preferred as the animal used. Rats and mice are more preferred, and balb/c mice are most preferred.

B lymphocytes are subsequently taken out of the immunized animal to carry out cell fusion with an animal cell having an infinite proliferation ability according to the method described in the above literature. A cell line preferred as an opponent of the fusion is a myeloma cell in a mouse, and P3-X63-Ag8-U1 myeloma cell (ATCC CRL1597) is especially preferred.

After the cell fusion, culturing is continued in a suitable selective medium according to a conventional method to sort out a cell line producing the antibody binding to the recombinant HAT from the survival cells by an ELISA method or the like. Cloning is further preferably conducted for the resulting cell producing the monoclonal antibody several times to sort out a strain having stability in monoclonality and antibody production. A limiting dilution method, a soft agarose method and a sorting method using a cell sorter or the like can be used as the cloning method.

An antibody having the activity is sorted out from the resulting monoclonal antibody against the recombinant HAT by, for example, the following method: That is, the recombinant HAT is added to a 96-well plate for the ELISA binding the anti-HAT monoclonal antibody with an antimurine IgG (Fc) antibody, and washing is carried out. A fluorescent substrate is then reacted to assay the HAT activity. Thereby, an antibody neutralizing the HAT activity can be screened. An enzymically labeled anti-HAT rabbit polyclonal antibody as a secondary antibody can be added to screen an antibody having high liquid-phase antigenic reactivity.

When the monoclonal antibody of the present invention is used for therapy of humans, a human type monoclonal antibody is preferably used. The human type monoclonal antibody includes the human monoclonal antibody and further the so-called chimeric antibody, i.e. the one having only a variable region comprising an amino acid sequence derived from an animal such as a mouse and a human amino acid sequence used in a constant region. In addition, a monoclonal antibody according to the so-called CDR grafting technique, i.e. the one having only a complementation determinant region comprising an amino acid sequence derived from an animal and all the other sequences derived from a human is also included in the human type monoclonal antibody herein described.

The chimeric antibody or the monoclonal antibody according to the CDR grafting technique can readily be prepared even by taking out the corresponding gene from a hybridoma of a mouse producing the monoclonal antibody of the present invention, recombining the resulting gene with a human antibody gene, introducing the recombinant into an animal cell and expressing the gene according to the present gene recombinant technique, for example, as described in Int. J. Cancer, 44, 424-433 (1989) and Pro. Natl. Acad. Sci. USA, 87, 8095-8099 (1990). The gene corresponding to the antibody of the present invention taken out of the hybridoma can be expressed in another animal cell for the purpose of improving the production efficiency of the monoclonal antibody without changing the amino acid sequence. The monoclonal antibody of the present invention includes a monoclonal antibody obtained by the above gene recombination technique and an animal cell producing the monoclonal antibody.

A method for cell fusion of a human B lymphocyte with a human or a mouse myeloma or a heteromyeloma is cited as the method for preparing the human type monoclonal antibody. The method for cell fusion with the human myeloma is as described in, for example, J. Immunol. Methods, 61, 17-32 (1983) and the method for cell fusion with the mouse myeloma is described in, for example, Pro. Natl. Acad. Sci. USA, 77, 6841-6845 (1980). The method for cell fusion with the heteromyeloma is described in, for example, Proc. Natl. Acad. Sci. USA, 81, 3214 (1984). The human B lymphocyte is preferably stimulated in vitro before the cell fusion when carrying out the methods. The method for stimulation may be conducted by the method described in, for example, Biochem Biophys Rec Commun, 135, 495 (1986).

In addition, transformation of the human B lymphocyte with an EB virus is cited as a method for preparing the human monoclonal antibody.

That is, the monoclonal antibody and polyclonal antibody of the present invention are antibodies capable of detecting and assaying the HAT present in vivo and in cells or antibodies capable of neutralizing the HAT activity or antibodies capable of inhibiting the activation of the HAT regardless of any kinds or those prepared by any methods or the like. Any of those skilled in the art may have no difficulty in the change of kinds or methods for preparation if the presence of the monoclonal antibody having the activity is known.

The monoclonal antibody and the polyclonal antibody of the present invention can be used for detecting and assaying the HAT. That is, the HAT concentration can be assayed by an immunochemical assay means known as the ELISA method or RIA method. For example, the HAT can be assayed with a high sensitivity by using a plate in which the anti-HAT monoclonal antibody is immobilized on the solid-phase side. The HAT can be detected and assayed by an immunohistostaining method, an immunocytostaining method, a flow cytometry or the like.

Furthermore, the HAT binding to a membrane can be assayed by using the monoclonal antibody and the polyclonal antibody of the present invention. The membrane-binding type HAT can be assayed by distinguishing an active type from an inactive type.

### Examples

The present invention is explained in more detail hereinafter by citing Examples, which are not intended to limit the present invention at all.

### [Example 1] Preparation of HAT

The purification of the natural HAT from the sputum was carried out according to the method disclosed in Japanese Unexamined Patent Publication No. 7-067640 and S. Yasuoka et al., Am. J. Respir. Cell Mol. Biol., 16, pp. 300-308, 1997. The purification of the recombinant HAT was conducted according to the method disclosed in Japanese Unexamined Patent Publication No. 8-89246, US Patent 5804410, EP 699763 and Yamaoka K. et al., J. Biol. Chem., 273(19), 11895-11901, 1998. Namely,, insect cells (Tn-5 cells) were grown to a density of 5 × 10⁶ cells/ml in a single layer, and the medium was removed. A serum-free medium containing a recombinant HAT expressed baculovirus (#1B3) was then added so as to provide an MOI = 0.2-0.5 based on the cell. Thereby, the insect cells were infected therewith and cultured for 3 days to express the HAT. The expressed protein was confirmed by the SDS-PAGE and the western blotting technique using an anti-HAT-N-19 peptide antibody (Anal. Biochem., 112, 195-203, 1981). The supernatant was separated from the cells by centrifugation (about 500 × g), and viruses were removed from the supernatant by ultrafiltration (Fuji Filter, Filtron Miniset, fractionating molecular weight: 300 kDa). The resultant supernatant was subsequently dialyzed or diluted with a 50 mM Tris hydrochloric acid-50 mM sodium chloride buffer (pH 8.0) overnight. The cells were suspended in a 50 mM Tris hydrochloric acid-500 mM sodium chloride buffer (pH 8.0), and Triton X-100 was added so as to provide the final concentration of 1%. The resulting suspension was allowed to stand at 0°C for 60 min to lyse the cells. The cell residue was centrifuged and then dialyzed or diluted in the 50 mM Tris hydrochloric acid-500 mM sodium chloride buffer (pH 8.0) overnight. The solutions were loaded into a benzamidine affinity column (manufactured by Pharmacia) equilibrated with a 50 mM Tris hydrochloric acid-500 mM Tris sodium chloride buffer (pH 8.0), washed with the same buffer and then eluted with a 10 mM hydrochloric acid-500 mM sodium chloride solution (pH 2.0). The trypsin-like protease activity was assayed and detected for each fraction by using the method described in Example 5 (1). The main peaks were collected to carry out SDS-PAGE, and a protein having a molecular weight of about 28 kDa was detected to confirm that the 28 kDa protein was the trypsin-like protease by a western blotting technique using the anti-trypsin-like protease peptide antibody [see Japanese Unexamined Patent Publication No. 8-89246, US Patent 5804410, EP 699763 and Yamaoka K. et al., J. Biol.Chem., 273(19), 11895-11901, 1998].

### [Example 2] Obtaining of anti-HAT polyclonal antibody

### (1) Obtaining of antipeptide antibody

A peptide (sequence No.1) of 20 residues, which has cycteine at the N-terminus of the sequence of the 1 to the 19 residues of a mature HAT, was chemically synthesized by a peptide synthesizer (Applied Biosystems, Model 431A). The synthesized peptide was dissolved in a 10 mM phosphoric acid buffer (pH 7.5) (10 mg/ml) and incubated with 10 mg of a maleimide-activated hemocyanin (manufactured by Boehringer Mannheim Biochemica) at 25°C for 2 hours, and the reaction solution was then dialyzed against a 10 mM phosphoric acid buffer (pH 7.5). The peptide bound to the hemocyanin was subcutaneously administered to rabbits (0.5 mg/a time). The administration was repeated 6 times at an interval of 2 weeks. The whole blood was collected to prepare serum, which was purified with a protein A Sepharose 4B column (manufactured by Pharmacia) to afford an anti-HAT polyclonal antibody (anti-N19 PAb).

### (2) Obtaining of anti-recombinant HAT antibody

The recombinant HAT prepared in Example 1 (40 *µ* g/a time), together with a complete Freund's adjuvant (manufactured by BACTO, 1:1), was intracutaneously administered to rabbits at an interval of 2 weeks. The operation was repeated four times, and whole blood was then collected to provide an antiserum. IgG was purified from the resulting serum with a protein A Sepharose 4B column (manufactured by Pharmacia) according to a conventional method to provide an anti-HAT polyclonal antibody (anti-rHAT PAb).

### [Example 3] Preparation of anti-HAT monoclonal antibody

### (1) Immunization of mice with recombinant HAT

The recombinant HAT prepared in Example 1 in an amount of 10-20 *µ* g/a time/head, together with a complete Freund's adjuvant (manufactured by BACTO, 1:1), was intraperitoneally administered to Balb/c mice (7-weeks-old, male) at an interval of 2 weeks. The operation was carried out four times, and 50 *µ*g of the recombinant HAT solution was intravenously administered before 3 days of cell fusion at the fifth time. Blood was collected from the tail vein of the mice, incubated at 37°C for 30 minutes and subsequently centrifuged at 3000 rpm for 10 minutes to collect serum. The anti-HAT antibody value in the murine serum was assayed with ELISA. The method is described hereinafter.

To a 96-well ELISA plate (Falcon 3912, manufactured by Becton Dickinson), was added 50 *µ*l of the recombinant HAT diluted to 1 *µ*g/ml with a 0.05 M Na₂CO₃ buffer (pH 10.5) . A reaction was carried out at 4°C overnight. The reaction product was then washed with PBS containing 0.05% of Tween 20 three times and then treated with PBS containing 3% of bovine serum albumin (BSA) at room temperature for 1 hour. The resulting treated product was washed with PBS containing 0.05% of Tween 20 three times, and 50 *µ*l of a specimen was subsequently added and reacted at room temperature for 1 hour. The reaction product was washed with PBS containing 0.05% of Tween 20 three times, and 50 *µ*l of an alkaline phosphatase-bound caprine antimurine IgG antibody (ZYMED) diluted 1000 times with PBS containing 3% of BSA was added and reacted at room temperature for 1 hour. The resulting reaction product was washed with PBS containing 0.05% of Tween 20 three times and then reacted with PNPP (p-nitrophenylphosphate, manufactured by Wako Pure Chemical Industries Ltd.) at room temperature for 1 hour to measure the absorbance at 405 nm.

### (2) Preparation of hybridoma by cell fusion

Mice were killed just before cell fusion, and spleen cells were homogenized in PBS. The residue was filtered through a nylon mesh and then subjected to centrifugal washing with PBS once. The cell fusion of the spleen cells with mouse myeloma cells (P3X63Ag8U.1) was performed according to a conventional method [Kohler, Miltstein; Nature, 256, 495-497 (1975)].

Namely, 5 ×10⁷ cells of the spleen cells and 5 ×10⁶ cells of the mouse myeloma cells P3×63Ag8U.1 (P3U1) were washed with RPMI 1640 medium and subsequently centrifuged at 1500 rpm for 5 minutes to provide cell pellets. One ml of a 35% polyethylene glycol solution (5.75 ml of RPMI 1640 medium + 3.5 ml of polyethylene glycol + 0.75 ml of dimethyl sulfoxide) was added for 2 minutes to gradually float the cells. One ml of RPMI 1640 medium was added for 2 minutes, and 2 ml of the RPMI 1640 medium was added for another 2 minutes. Four ml of a GIT-HAT medium (GIT medium containing 95 µM of hypoxanthine, 0.4 µM of aminopterin, 1.6 µM of thymidine and 5% of FCS) was then added for 2 minutes. Furthermore, 8 ml of the GIT-HAT medium was subsequently added for another 2 minutes. Incubation was conducted at 37°C for 30 minutes, and the resulting mixture was then dispensed to one 96-well flat bottom plate seeded with about 10⁴ murine peritoneal exudate cells per well and cultured at 37°C in the presence of 5% of CO₂.

After one week, a half of the medium was exchanged with a GIT-HT medium (medium after removing the aminopterin from the GIT-HAT medium), and culturing was conducted at 37°C in the presence of 5% of CO₂ for about one week to thereby afford several hybridoma colonies per well.

### (3) Screening of hybridoma

After two weeks, screening was carried out by using a plate coated with the recombinant HAT. A 50 mM Na₂CO₃ buffer (pH 10.5) solution (1 *µ*g/ml) of the recombinant HAT by a volume of 50 *µ*l for each well was dispensed to the 96-well plate (manufactured by Falcon, made of PVC) and allowed to stand at 4°C overnight. After washing, a 3% BSA/PBS in a volume of 200 *µ*l for each well was added to carry out blocking at 37°C for 1 hour. After rewashing, a culture supernatant in a volume of 50 *µ*l for each well was added and allowed to stand at room temperature for 1 hour. Washing was subsequently conducted with 0.05% Tween/PBS three times.

A caprine antimurine IgG-alkaline phosphatase conjugate (Tago) diluted 2000 times with PBS containing 3% of BSA and 0.2% of skimmilk in a volume of 50 *µ*l for each well was subsequently added and allowed to stand at room temperature for 1 hour. Rewashing was carried out, and a 1 mg/ml solution of p-nitrophenylphosphate disodium salt (Wako Pure Chemical Industries Ltd.) dissolved in a 1 M diethanolamine buffer (pH 9.8) containing 0.25 mM of magnesim chloride in a volume of 100 *µ*l for each well was added and reacted at room temperature for 30 minutes. The absorbance thereof at 405 nm was examined with an ELISA reader measuring instrument (Molecular Davice Vmax) for the 96-well plate to select a hybridoma secreting a monoclonal antibody binding to the recombinant HAT.

### (4) Cloning of hybridoma

The selected hybridoma was cloned according to a limiting dilution method twice and established. Specifically, a preparation of murine peritoneal exudate cells obtained in a ratio of 10⁶ cells/ml in the HT medium was dispensed to each well, and hybridoma cells suspended in the HT medium at a ratio of 0.5 cell per well were seeded thereinto. Culturing was conducted in a CO₂ incubator at 5% of CO₂ and at 37°C for 2 weeks. The resultant culture supernatant was screened according to the above ELISA method and established by picking up a single colony.

### (5) Culturing of hybridoma and purification of antibody

Six clones in the 20 clones producing the anti-HAT antibody obtained by the above operation were adapted to an eRDF medium which was a serum-free medium containing insulin, transferrin, ethanolamine and a selenite (Gibco BRL) and cultured to recover the culture supernatant. Antibodies in the culture supernatant were purified with a protein G column according to a conventional method.

### (6) Subtyping of HAT monoclonal antibody

Subtyping was conducted for the 6 clones of the purified anti-HAT monoclonal antibody obtained according to the above operation (5) by using IsoStrip (Isotyping Kit of the murine monoclonal antibody) manufactured by Boehringer. As a result, it was found that 3 clones of #A3, #B3 and #B5 were IgG1, and #A6 and #C2 were IgM. The clone #C3 was unknown.

### [Example 4] Western blotting with anti-HAT antibody

The recombinant HAT or natural HAT was fractionated under reducing conditions by an SDS polyacrylmide gel electrophoresis, and western blocking for a nitrocellulose membrane (manufactured by BIO-RAD) was then carried out. The resulting substance was reacted with the anti-HAT antibody which was obtained in [Examples 2 and 3] by treatment with PBS containing 3% of BSA at room temperature overnight and then diluted to 10 *µ*g/ml with the PBS containing 3% of the BSA at room temperature for 1 hour. The nitrocellulose membrane was washed with the PBS containing 1% of the BSA 6 times and subsequently reacted with an alkaline phosphatase-bound caprine antihuman IgG antibody (TAGO) diluted to 25 *µ*g/ml with the PBS containing 3% of the BSA at room temperature for 1 hour. The nitrocellulose membrane was washed with the PBS containing 1% of the BSA 6 times and then reacted with NBT (Nitro-Blue Tetrazolium Chloride)/BICP (5-Bromo-4-Chloro-3'-Indolyphosphate p-Toluidine Salt, PIERCE) at room temperature for 10 minutes to develop colors. Thereby, it was found that the respective anti-HAT antibodies were bound to the recombinant HAT. The reactivity of the western blotting was high in the case of #A3 and #B5 and extremely low in the case of #B3 in the monoclonal antibody. As for the polyclonal antibody, the reactivity was high in the case of the anti-N-19 antibody (anti-N19 PAb) and low in the case of the anti-rHAT antibody (anti-rHAT PAb). Furthermore, the anti-rHAT PAb manifested no cross-reactivity with tryptase and trypsin derived from a mast cell. Furthermore, normal rabbit IgG was used as a negative control.

### [Example 5] Evaluation of HAT neutralizing activity

### (1) Assay of HAT activity

To 1.0 ml of a 50 mM Tris-HCl buffer (pH 8.6) containing 100 *µ*M of a synthetic substrate for trypsin Boc-Phe-Ser-Arg-MCA (MCA: Methylcoumarin amide) and 0.01% of BSA, was added 50 *µ*l of an HAT-containing solution. Incubation was carried out at 37°C for 1 hour. One ml of a 30% acetic acid was then added, and the amount of the produced 7-amino-4-methylcoumarin (AMC) was determined by a fluorophotometry (fluorescence 460 nm and excitation light 380 nm) to calculate the activity of the enzyme. The activity of producing 1 pM of the AMC for 1 minute was defined as 1 unit (1 unit = 1 pM AMC/minute).

### (2) Evaluation of HAT neutralizing activity of anti-HAT antibody

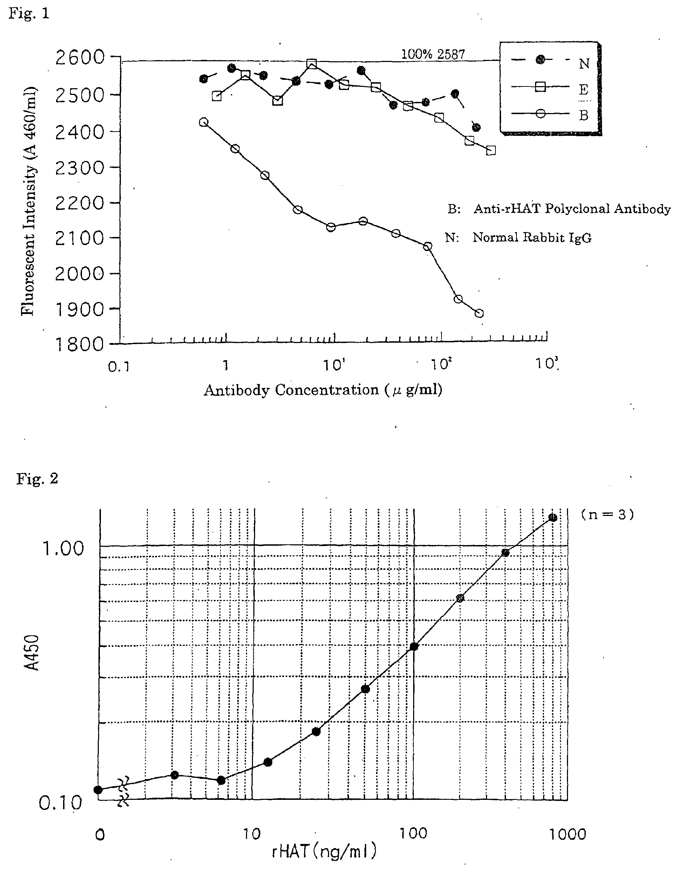

To 0.5 ml of a 50 mM Tris-HCl buffer (pH 8.6) containing 2 ng/ml of the recombinant HAT and 0.01% of BSA, was added 50 *µ*l of an anti-HAT antibody-containing solution. Incubation was conducted at 37 °C for 30 minutes, and 0.5 ml of a 50 mM Tris-HCl buffer (pH 8.6) containing 200 *µ*M of the synthetic substrate for trypsin Boc-Phe-Ser-Arg-MCA was then added. Incubation was carried out at 37°C for 1 hour. One ml of a 30% acetic acid was subsequently added, and the amount of the produced 7-amino-4-methylcoumarin (AMC) was determined by a fluorophotometry (fluorescence 460 nm and excitation light 380 nm) to evaluate the enzyme activity neutralizing ability of the anti-HAT antibody. Fig. 1 shows the obtained results. Activity inhibitory effects of 27% were recognized in the anti-HAT polyclonal antibody obtained by immunizing the recombinant HAT (insect cell-baculovirus system) obtained in Example 2 at a concentration of 236 *µ*g/ml.

### [Example 6] Construction of HAT assaying system

### 1. Construction of sandwich EIA (Enzyme Immunoassay) with polyclonal antibody

The sandwich EIA system was constructed by performing the following operations, using the anti-HAT polyclonal antibody (anti-rHAT PAb) obtained in Example 2(B).

### (A) Preparation of horseradish peroxidase (HRP)-labeled antibody

### (1) Preparation of antibody (F(ab')₂);

To 1 ml of a PBS solution containing 2.0 mg/ml of the antibody (IgG), were added 100 *µ*l of a 1 M acetic acid buffer (pH 4.2) and a solution prepared by dissolving 40 *µ*g of pepsin in 20 *µ*l of the same buffer. A reaction was carried out at 37°C for 4 hours. After completing the reaction, the reaction product was separated with a Sephadex G25 column (φ 2 cm × 45 cm) equilibrated with PBS to collect the F(ab')₂.

### (2) HRP labeling of antibody (F(ab')₂)

To 2 ml of a 0.15 M NaCl (pH 7.4) solution of a 0.01 M phosphoric acid containing 1 mg/ml of the antibody (F(ab')₂ ), was added 50 *µ*l of a 10 mg/ml dimethylformamide solution of N-(m-maleimidobenzoic acid)-N-succinimide ester (MBS). A reaction was carried out at 25°C for 30 minutes. The maleimidated antibody and the unreacted MBS were separated, by carrying out gel filtration with a 0.1 M phosphoric acid buffer (pH 6.0), using a column filled with Sephadex G-25. On the other hand, 120 *µ*l of a 60 mg/ml dimethylformamide solution of S-acetylmercaptosuccinic anhydride was added to 2 ml of a 0.1 M phosphoric acid buffer (pH 6.5) of 10 mg/ml of HRP and reacted at 25°C for 2 hours. To the reaction mixture, were added 800 *µ*l of a 0.1 M Tris-hydrochloric acid buffer (pH 7.0), 160 *µ*l of 0.1 M EDTA and 1.6 ml of 1 M hydroxylamine. A reaction was carried out at 0°C for 4 minutes. The reaction solution was then placed in a collodion bag and dialyzed at 4°C for 3 days by using a solution of a 0.1 M phosphoric acid buffer (pH 6.0) containing 5 mM of EDTA to provide a thiolated HRP. Two mg of the maleimidated antibody was then mixed with 4 mg of the thiolated HRP, and the mixture was concentrated with a collodion bag under cooling with ice until a 4-10 mg/ml protein concentration was reached, and the concentrate was allowed to stand at 15-20°C overnight. The resulting liquid was subjected to gel filtration with a column filled with Ultrogel AcA44 (Manufactured by Pharmacia-LKB) to afford an anti-HAT (F(ab')₂) HRP-labeled antibody.

### (B) Preparation of antibody-immobilized plate

A 96-well plate (manufactured by Sumitomo Bakelite Co., Ltd., MS-3796F/Carboplate) was sufficiently washed, then allowed to stand in a 20 µg/ml PBS solution of the antibody at 4°C a whole day and night, subsequently washed with the PBS and then allowed to stand in a PBS solution of 1% bovine serum albumin (BSA) at 4°C a whole day and night to provide an antibody-immobilized plate.

### (C) Construction of assaying system

To the anti-HAT antibody IgG-immobilized plate prepared in the above (B), was added 100 *µ*l of a 1% BSA-0.1% skimmilk-containing 10 mM PBS (pH 7.2) solution (diluted specimen liquid) containing a purified recombinant HAT (standard substance) within the range of 0 to1600 ng/ml. Incubation was carried out at 25°C for 4 hours. The resulting mixture was then washed with a 10 mM PBS (pH 7.2) containing 0.05% of Tween 20, and a solution of the anti-HAT (F(ab')₂) HRP-labeled antibody (500 *µ*g/ml) prepared in the above (A) and diluted 100 times with a 10 mM PBS (pH 7.2) containing 1% of BSA by a volume of 100 *µ*l was added to each well. Incubation was conducted at 25°C for 2 hours. The solution in each well was removed by suction, and washing was subsequently performed with a 10 mM PBS (pH 7.2) containing 0.05% of Tween 20. A 0.1 M phosphoric acid/citric acid buffer (pH 4.3) containing 0.02% of 3,3',5,5'-tetramethylbenzidine hydrochloride and 2.5 mM of H₂O₂ by a volume of 100 *µ*l was added to each well and reacted at 25°C for 30 minutes. A 0.5 M aqueous solution of sulfuric acid as a reaction terminator by a volume of 100 µl was then added to stop the enzyme reaction. The absorption intensity of the solution at a wavelength of 450 nm was then measured by using a spectrophotometer and plotted versus a standard substance concentration of 0 to 1600 ng/ml. Fig. 2 shows the obtained results.

### (D) Detection of HAT in components in vivo

### (1) Detection of HAT in saliva

The assaying system in the above (C) was used to assay the HAT in human saliva. The saliva collected from humans were centrifuged at 15000 rpm for 15 minutes to provide a supernatant, which was used as an assaying sample. Assay was made for the total 6 specimens of the intact stock solution and solutions diluted 16 times with a 1% BSA-0.1% skimmilk-containing 10 mM PBS (pH 7.2) solution (diluted specimen liquid). As for three specimens, the values of the stock solution were adopted because assayed values were low. As for other three specimens, the values of the solutions diluted 16 times were adopted to provide the HAT concentrations in the saliva. Table 1 shows the results of assay together with the HAT activity (trypsin substrate hydrolytic activity) in the specimens.

**Table 1**

| | HAT (ng/ml) | |
|---|---|---|
| Salivary Specimen | ELISA | Expressed in Terms of HAT Activity* |
| KM123 | 1616 | 3333 |
| KY123 | 24 | 120 |
| KT123 | 22 | 518 |
| HO123 | 720 | 1772 |
| KY112 | 25 | 208 |
| HO112 | 784 | 2349 |

| | | |
|---|---|---|
| *HAT activity expressed in terms of recombinant HAT | | |

### (2) Detection of HAT in sputum

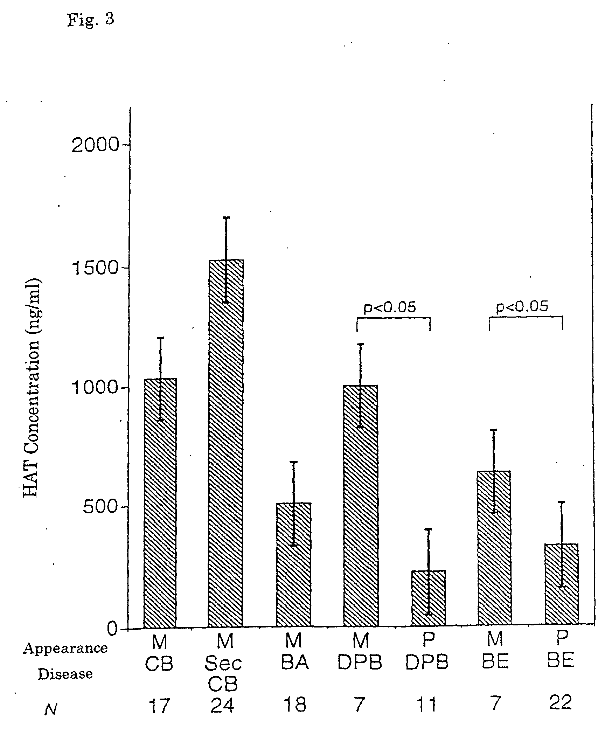

The sputum was collected from patients suffering from chronic airway inflammations, diluted with a physiological saline in a volume of 9 times, then homogenized and centrifuged at 10,000 × g for 10 minutes to provide the supernatant as assay specimens, which were diluted 20 times to carry out assay. Fig. 3 shows the obtained results. Mucous-mucopurulent sputum was collected from the following patients:

| | |
|---|---|
| CB | Chronic bronchitis |
| SecCB | Secondary bronchitis |
| BA | Bronchial asthma |
| BE | Bronchiectasis |
| DPB | Diffuse panbronchiolitis |

Furthermore, purulent sputum was collected from the following patients:

| | |
|---|---|
| BE | Bronchiectasis |
| DPB | Diffuse panbronchiolitis |

In Fig. 3, M indicates the mucous sputum, and P indicates the mucopurulent sputum.

The purulent sputum shows lower assayed values than those of the mucous sputum.

### 2. Construction of sandwich EIA with monoclonal antibody and polyclonal antibody

### (1) Evaluation of liquid-phase antigenic reactivity of each anti-HAT monoclonal antibody

Evaluation of binding properties to the recombinant HAT in the liquid phase was made for three kinds of the anti-HAT monoclonal antibodies #A3, #B3 and #B5 which were IgG1 type by subtyping.

The method for evaluation was carried out according to 1. (B) and (C) of Example 6. Namely, a 1 *µ*g/ml or a 5 *µ*g/ml PBS solution of each anti-HAT monoclonal antibody in a volume of 50 *µ*l/well was added to a 96-well plate and allowed to stand at 4°C overnight to immobilize the antibodies in the plate.

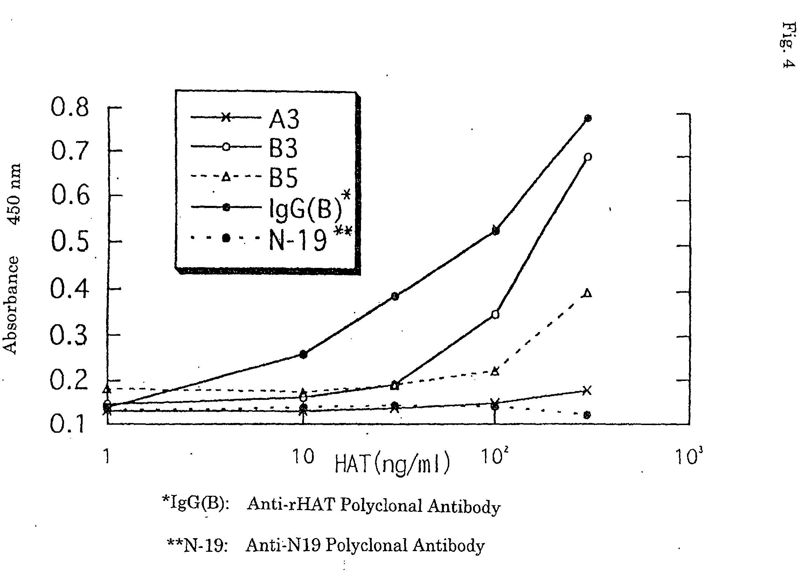

A 1% BSA-0.1% skimmilk-containing 10 mM PBS (pH 7.2) solution (diluted specimen liquid) containing the purified recombinant HAT (standard substance) within the range of 0 to 300 ng/ml in a volume of 100 *µ*l was added to the prepared anti-HAT monoclonal antibody-immobilized plate and incubated at 25°C for 4 hours. Washing was then carried out with a 10 mM PBS (pH 7.2) containing 0.05% of Tween 20, and a solution of the HRP-labeled antibody (500 *µ*g/ml) which was prepared in (A) and diluted to 100 times with a 1% BSA-containing 10 mM PBS (pH 7.2) by a volume of 100 µl was added to each well and incubated at 25°C for 2 hours. The solution in each well was removed by suction, and washing was subsequently conducted with a 10 mM PBS (pH 7.2) containing 0.05% of Tween 20. A 0.1 M phosphoric acid/citric acid buffer (pH 4.3) containing 0.02% of 3,3',5,5'-tetramethylbenzidine hydrochloride and 2.5 mM of H₂O₂ by a volume of 100 *µ*l was added to each well and reacted at 25°C for 30 minutes, and a 0.5 M aqueous solution of sulfuric acid as a reaction terminator by a volume of 100 µl was added to stop the enzyme reaction. The absorption intensity of the solution was measured at a wavelength of 450 nm by using a spectrophotometer and plotted verses a standard substance concentration of 0 to 300 ng/ml. As a result, it was made clear that the liquid-phase antigenic reactivity was higher in the order of #B3>#B5>#A3. Fig. 4 shows the obtained results.

### (2) Construction of assaying system

### (A) Preparation of biotinylated antibody

The biotinylation of the IgG was conducted as follows by using the #B3 anti-HAT monoclonal antibody having the highest liquid-phase antigenic reactivity in the evaluation results in the above (1) and the anti-HAT polyclonal antibody (anti-rHAT PAb) obtained in Example 2(B): One ml of 1-3 mg/ml IgG solutions (PBS(-)) was mixed with an equal volume of a 0.2 M NaHCO₃ (pH 8.0), and 10 mM of a biotinylating reagent at a molar concentration of 100 times was then added. Incubation was carried out at 37°C for 1 hour. The biotinylating reagent was prepared by dissolving NHS-LC-Biotin (manufactured by PIERCE, #21335: molecular weight 556) in 10 mM with dimethylformamide. A 1M monoethanolamine in a volume of 1/10 was then added, and incubation was further conducted at 37°C for 1 hour. The volume of the reaction solution was increased to 2.5 ml with PBS(-) using a graduate, and the resulting solution was applied to a G-25 gel filtration column and discharged with the PBS(-) to recover a biotinylated IgG. Furthermore, BSA at the final concentration of 3% and NaN₃ at the final concentration of 0.1% were added thereto, and the resulting mixture was preserved.

### (B) Preparation of antibody-immobilized plate

A 96-well plate (manufactured by Sumitomo Bakelite Co., Ltd., MS-3796F/Carboplate) was sufficiently washed, and a 20 *µ*g/ml PBS solution of the antibodies (#B3 and anti-rHAT PAb) by a volume of 100 *µ*l was added to each well and allowed to stand at a temperature of 4°C a whole day and night. The well was washed with a PBS-0.05% Tween 20, and 380 *µ*l of a PBS solution of a 1% bovine serum albumin (BSA) was added to each well and allowed to stand at 4°C a whole day and night to provide an antibody-immobilized plate.

### (C) Construction of assaying system

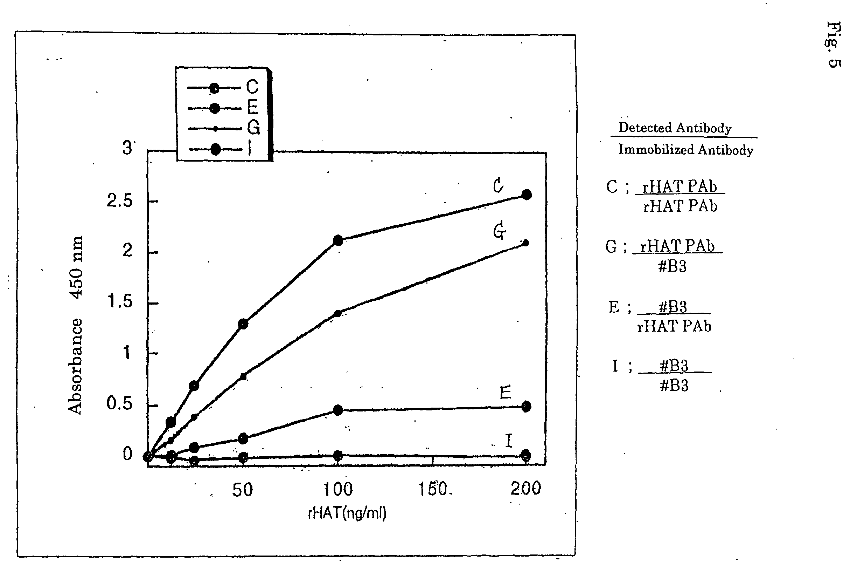

To the anti-HAT IgG-immobilized plate prepared in the above (B), were added 50 *µ*l of a 1% BSA-0.1% skimmilk-containing 10 mM PBS (pH 7.2) solution (diluted specimen solution) containing the purified recombinant HAT (standard substance) within the range of 0 to 400 ng/ml and 50 *µ*l of 1% BSA-0.1% skimmilk-containing 10 mM PBS (pH 7.2) solution containing 10 *µ*g/ml of the biotinylated IgG prepared in the above (A). The resulting mixture was allowed to stand at 4°C overnight. The plate was subsequently washed, and a 1% BSA-0.1% skimmilk-containing 10 mM PBS (pH 7.2) solution prepared by diluting a streptavidin-labeled peroxidase (manufactured by TAGO, #SNN1004) to 10000 times in a volume of 100 *µ*l/well was then added. Incubation was conducted at 25°C for 1 hour. The solution in each well was subsequently removed by suction, and washing was then carried out with a 10 mM PBS (pH 7.2) containing 0.05% of Tween 20. A 0.1 M phosphoric acid/citric acid buffer (pH 4.3) containing 0.02% of 3,3',5,5'-tetramethylbenzidine hydrochloride and 2.5 mM of H₂O₂ by a volume of 100 *µ*l was added to each well and reacted at 25°C for 15 minutes. A 0.5 M aqueous solution of sulfuric acid as a reaction terminator by a volume of 100 *µ*l was subsequently added to stop the enzyme reaction. The absorption intensity at a wavelength of 450 nm was measured by using a spectrophotometer and plotted versus a standard substance concentration of 0 to 200 ng/ml. Fig. 5 shows the obtained results.

### Possibility of Industrial Utilization

The monoclonal antibody and polyclonal antibody of the present invention are used as a therapeutic agent to thereby suppress or modify physiological actions such as actions of the HAT on the coagulating and fibrinolytic system, actions on airway remodeling, actions on airway inflammations, actions on the proliferation or metastasis of cancers, actions on mucociliary movement or antiviral infectious actions and can be used for ameliorating or treating the morbid state.

Furthermore, the HAT concentration in, for example, saliva, sputum and blood can be assayed with a diagnostic agent using the monoclonal antibody and polyclonal antibody of the present invention.

## Claims

1. An antibody binding to a human airway trypsin-like protease.

2. The antibody as set forth in claim 1, wherein the antibody is a monoclonal antibody.

3. The antibody as set forth in claim 2, wherein the antibody is a murine monoclonal antibody.

4. The antibody as set forth in claim 3, wherein the antibody is a murine IgG monoclonal antibody.

5. The antibody as set forth in claim 3, wherein the antibody is a murine IgM monoclonal antibody.

6. The antibody as set forth in claim 1, wherein the antibody is a polyclonal antibody.

7. The antibody as set forth in claim 6, wherein the antibody is a rabbit polyclonal antibody.

8. An antibody obtained by immunizing a partial peptide of a human airway trypsin-like protease.

9. An antibody obtained by immunizing a partial peptide containing a part or all of 1 to 19 residues on the N-terminal side of a mature part of a human airway trypsin like protease.

10. The antibody as set forth in claim 8 or 9, wherein the antibody is a polyclonal antibody.

11. The antibody as set forth in claim 10, wherein the antibody is a rabbit polyclonal antibody.

12. The antibody as set forth in any of claims 1 to 11, wherein a natural human airway trypsin-like protease in vivo can be assayed by an enzyme immunoassay using the same.

13. The antibody as set forth in any of claims 1 to 11, wherein a natural human airway trypsin-like protease in vivo can be detected according to a western blotting technique using the same.

14. The antibody as set forth in any of claims 1 to 11, wherein the activation of the human airway trypsin-like protease is inhibited.

15. The antibody as set forth in any of claims 1 to 11, wherein the human airway trypsin-like protease activity is neutralized.

16. The antibody as set forth in any of claims 3 to 5, wherein the epitope is present between the 187th and the 418th positions of the amino acid sequence of the human airway trypsin-like protease.

17. The antibody as set forth in any of claims 1 to 11, wherein an immunogen is a recombinant human airway trypsin-like protease.

18. A method for detecting or assaying the human airway trypsin-like protease by using the antibody as set forth in any of claims 1 to 11.

19. An enzyme immunoassay for the human airway trypsin-like protease using the antibody as set forth in any of claims 1 to 11.

20. A therapeutic agent comprising the antibody as set forth in claim 14.

21. A therapeutic agent comprising the antibody as set forth in claim 15.

22. An animal cell producing the antibody as set forth in any of claims 2 to 5.

23. A hybdridoma cell producing the antibody as set forth in any of claims 2 to 5 or an animal cell derived from the hybridoma cell.
